(19) **Europäisches Patentamt European Patent Office Office européen des brevets**

(11) **EP 1 484 083 B1**

(12) **EUROPEAN PATENT SPECIFICATION**

(45) Date of publication and mention of the grant of the patent:
**23.05.2007 Bulletin 2007/21**

(51) Int Cl.:
*A61N 1/00* *(2006.01)*  *A61N 1/365* *(2006.01)*

(21) Application number: **04253249.9**

(22) Date of filing: **01.06.2004**

(54) **Physiologic stimulator tuning apparatus**

Gerät zur Einstellung einer physiologischen Stimulation

Appareil de réglage de stimulateur physiologique

(84) Designated Contracting States:
**AT BE BG CH CY CZ DE DK EE ES FI FR GB GR HU IE IT LI LU MC NL PL PT RO SE SI SK TR**

(30) Priority: **02.06.2003 US 453820**

(43) Date of publication of application:
**08.12.2004 Bulletin 2004/50**

(73) Proprietor: **CardioDynamics International Corporation**
**San Diego,**
**California 92121 (US)**

(72) Inventors:
• **Hepp, Dennis G.**
**Minnesota 55433 (US)**
• **Baura, Gail D.**
**San Diego, California 92107 (US)**
• **Malecha, Jeremy R.**
**San Diego, California 92109 (US)**

(74) Representative: **Fitchett, Stuart Paul**
**Saunders & Dolleymore,**
**9 Rickmansworth Road**
**Watford,**
**Hertfordshire WD18 0JU (GB)**

(56) References cited:
WO-A-01/24875    US-A- 4 901 726
US-A- 5 999 854    US-A- 6 055 454
US-B1- 6 263 243

**Description**

Background of the Invention

**1. Field of the Invention**

The present invention relates generally to the field of biomedical treatment and analysis, and particularly to an improved apparatus and method for tuning and operating stimulation devices such as pacemakers.

**2. Description of Related Technology**

Physiologic stimulation devices are well known in the medical arts. These devices are used to, *inter alia,* provide regular or incidental stimulation to the tissue (e.g., muscle or cardiac tissue) of a living subject, typically using electrical current, in order to induce a response in that tissue, such as a contraction of the tissue. One such well known device is the implantable cardiac pacemaker.

A pacemaker is a device that generally consists of a pulse generator, electrical power source (e.g., battery), and leads or electrodes for passing the generated pulse(s) to the subject's tissue. It produces voltage impulses of short duration to the endocardium or myocardium (heart). In the human being, the electrical leads may be positioned in the right ventricle only (so-called "single chamber" pacing); the right atrium and right ventricle ("dual chamber" pacing); or the right atrium, right ventricle, and left ventricle ("biventricular" pacing). When properly applied, an artificial pacing voltage spike generated by the pacemaker excites the relevant cardiac tissue by the creation of an electrical field at the interface of the stimulating electrode and the underlying tissue. This excitation initiates a self-regenerating wavefront of action potentials that propagate away from the site of stimulation, leading to contraction of the desired heart chamber (right atrial/ventricular stimulation causes left atrial/ventricular contraction), or coordination of chamber contraction (left and right ventricle interaction).

Typically, a pacemaker is implanted to compensate for a conduction defect that prevents an action potential in the sinoatrial node from being efficiently or completely transmitted to the atrioventricular node, to the His bundle, and to left and right the bundle branches. A permanent pacemaker may also be indicated for neurally mediated syncope (fainting) and cardiomyopathy (heart muscle disease).

After implantation, the parameter settings of a pacemaker may be customized from the default settings provided with the device using a pacemaker programmer. A pacemaker programmer enables both programmability and telemetry of programming commands, administrative data, programmed data, measured data, and diagnostic data. Commands and data are typically transmitted across through an electromagnetic telemetry interface, usually in the form of a hand-held wand or paddle, which is positioned proximate to the pacemaker pulse generator, external to the subject. Communication between the implanted device and the programming device may be continuous, or may require a programmer command to initiate programmer transmission to the pulse generator. U. S. Patent No. 5,891,178 to Mann, et al. issued April 6, 1999 and entitled "Programmer system and associated methods for rapidly evaluating and programming an implanted cardiac device" describes one such pacemaker programmer and telemetry interface.

One of the most important pacemaker parameters that may be optimized is the atrioventricular (AV) delay, or the time interval between right atrial and right ventricular stimulation. For biventricular pacing, another important parameter is biventricular (VV) skew, the time interval between right and left ventricular stimulation and the order of stimulation. During pacemaker tuning, various trial parameter settings are typically programmed and evaluated. The parameter setting that produces the highest left ventricular stroke volume is considered the optimal parameter setting. Typically, echocardiography techniques are used as the means and reference standard for determining left ventricular stroke volume.

However, the aforementioned technique of echocardiographic tuning can be clinically time consuming, and is not reimbursed by Medicare.

*Impedance Cardiography*

As is well known, noninvasive estimates of cardiac output (CO) can be obtained using impedance cardiography (ICG). Strictly speaking, impedance cardiography, also known as thoracic bioimpedance or impedance plethysmography, is used to measure the stroke volume (SV) of the heart. As shown in Eqn. (1), when the stroke volume is multiplied by heart rate, cardiac output is obtained.

$$CO = stroke\ volume \times heart\ rate. \qquad (1)$$

The heart rate is obtained from an electrocardiogram (ECG). The basic method of correlating thoracic, or chest cavity, impedance, $Z_T(t)$, with stroke volume was developed by Kubicek, et al. at the University of Minnesota for use by NASA. See, e.g., U.S. Reissue Patent No. 30,101 entitled "Impedance plethysmograph" issued Sept. 25, 1979. The method generally comprises modeling the thoracic impedance $Z_T(t)$ as a constant impedance, $Z_o$, and time-varying impedance, $\Delta Z(t)$. The time-varying impedance is measured by way of an impedance waveform derived from electrodes placed on various locations of the subject's thorax; changes in the impedance over time can then be related to the change in fluidic volume (i.e., stroke volume), and ultimately cardiac output via Eqn. (1) above.

More recently, further improvements have been made to the basic ICG technique, to include highly accurate methods of compensating for physiologic effects resulting from the anatomy of particular individual subjects, identifying fiducial points and other relevant artifacts in the ICG waveform (as well as corresponding ECG waveforms), signal processing and analysis of the data, and coordination of the information gained via ICG with other parametric measurements. See, e.g., U.S. Patent No. 6,561,986 to Baura, et al. issued May 13, 2003 and entitled "Method and apparatus for hemodynamic assessment including fiducial point detection" (teaching, *inter alia,* improved methods and apparatus for fiducial point detection in the context of ICG and ECG waveforms), as well as co-pending U.S. patent application Serial Nos. 09/613,183 entitled "Apparatus And Method For Determining Cardiac Output In A Living Subject" filed July 10, 2000, now U.S. Patent No. 6,636,754 issued October 21, 2003, (teaching, *inter alia,* improved ICG apparatus and methods based on predetermined electrode spacing), and 09/903,473 entitled "Apparatus and Method for Determining Cardiac Output in a Living Subject" filed July 10, 2001, now U.S. Patent No. 6,602,201 issued August 5, 2003 (teaching, *inter alia,* an improved ICG module architecture and signal processing).

However, even in its most basic form, ICG has been proven to be a rapid, effective, and easily administered non-invasive technique for determining the volumetric output of the heart of the human being (or other species). Document EP-A-1284149 describes an ambulatory recording device comprising a microcontroller which is adapted to determine various markers and state conditions based on the heart and patient sensor signals (figure 5).

Based on the foregoing, there is a need for an improved apparatus for non-invasively tuning and evaluating implantable stimulation devices (such as cardiac pacemakers). Such improved apparatus ideally would allow the clinician to rapidly and accurately tune one or more parameters associated with the stimulation device, including e.g., the aforementioned AV and VV parameters as appropriate. Additionally, the improved apparatus would also permit such tuning and operation by relatively unqualified personnel (to prospectively include the subject themselves).

Summary of the Invention

The present invention satisfies the aforementioned needs by providing an improved apparatus for tuning and operating an implantable stimulation device used in conjunction with a living subject.

In an aspect of the invention, an improved evaluation apparatus for use with a cardiac stimulation device is disclosed. The apparatus generally comprises impedance cardiography apparatus adapted for determining at least one aspect of cardiac function under varied conditions of stimulation produced by the stimulation device. In one exemplary embodiment, the apparatus comprises an ICG module coupled to a pacemaker control apparatus, the latter adapted to control the conditions of said stimulation device through an electromagnetic or inductive interface. The evaluation apparatus is further configured to initiate and perform evaluation and tuning through a single user action; e.g., the press of a single button.

In an aspect of the invention, an improved cardiac evaluation apparatus is disclosed. The apparatus generally comprises: a stimulation source adapted to produce a stimulation current; a plurality of electrodes adapted for use on a living subject, at least a portion of the electrodes being further adapted to apply the stimulation current to the subject; a data interface adapted to transmit data between the apparatus and a pacemaker controller configured to control at least one parameter associated with a pacemaker; and a signal processing apparatus operatively coupled to at least a portion of the electrodes and the data interface, the signal processing apparatus adapted to coordinate evaluation of signals obtained from the at least portion of electrodes with changes in the at least one parameter of the pacemaker in order to identify an optimal value thereof.

Brief Description of the Drawings

Fig. 1 is a logical flow diagram illustrating one exemplary embodiment of the method of tuning a stimulation device disposed within a living subject according to the invention.

Fig. 1a is a plan view of a typical human thorax illustrating an exemplary placement of the ICG electrode arrays of the present invention during ICG analysis in support of tuning.

Fig. 1b is a logical flow diagram illustrating one exemplary embodiment of the method of optimizing the placement of leads associated with a stimulation device disposed within a living subject according to the invention.

Fig. 2 is a functional block diagram illustrating one exemplary embodiment of the tuning apparatus of the present invention.

Fig. 3 is a functional block diagram illustrating a second exemplary embodiment of the tuning apparatus of the present invention, wherein the ICG and programmer functionality are substantially integrated into a common device.

Detailed Description of the Invention

Reference is now made to the drawings wherein like numerals refer to like parts throughout.

It is noted that while the invention is described herein in terms of an apparatus for determining stroke volume and tuning a stimulation device, suitable for use on the thorax of a human subject, the invention may also conceivably be embodied or adapted to other locations on the body and/or other warm-blooded species. All such adaptations and alternate embodiments are considered to fall within the scope of the claims appended hereto.

As used herein, the term "digital processor" is meant generally to include all types of digital processing devices including, without limitation, digital signal processors (DSPs), reduced instruction set computers (RISC), general-purpose (CISC) processors, microprocessors, and application-specific integrated circuits (ASICs). Such digital processors may be contained on a single unitary IC die, or distributed across multiple components.

As used herein, the terms "monitor" and "monitoring device" are used generally to refer to devices adapted to perform monitoring, display, user interface, and/or control functions. Such devices may be dedicated to a particular function, or multi-purpose devices adaptable to performing a variety of functions and/or interfacing with a number of functional modules.

As used herein, the terms "stimulation device" and "stimulator" refer generally to devices which may be used to stimulate living tissue or muscle to obtain a desired response. One exemplary stimulator is the cardiac pacemaker, although it will be recognized that the present invention is not limited to traditional cardiac pacemaker devices alone. For example, another exemplary stimulator comprises the implantable cardiovertor-defibrillator, which always incorporates single chamber pacemaker functionality and often dual chamber or biventricular pacing..

*Overview*

In a broad sense, the present invention provides improved apparatus for tuning and operating an implantable stimulation device (e.g., cardiac pacemaker). These improved apparatuses are based on the use of ICG technologies (as compared to the prior art echocardiography tuning methods, see Kindermann, et al., *PACE,* 20[Pt. I}:2453-2462, 1997), such ICG technologies enabling more rapid pacemaker tuning, with equivalent or even increased accuracy.

In the exemplary embodiment, the use of ICG techniques to perform the aforementioned tuning is highly automated to the point where the user or clinician can perform the entire tuning process with little more than the selection of one function (or push of one "button"). This greatly simplifies the tuning process over the prior art methods, the latter being necessarily more complex and time consuming. Accordingly, such simplified tuning as offered by the present invention opens up the field of prospective users/operators to even those having little skill or experience in performing such optimizations, including the patient being "tuned" himself.

*Methodology*

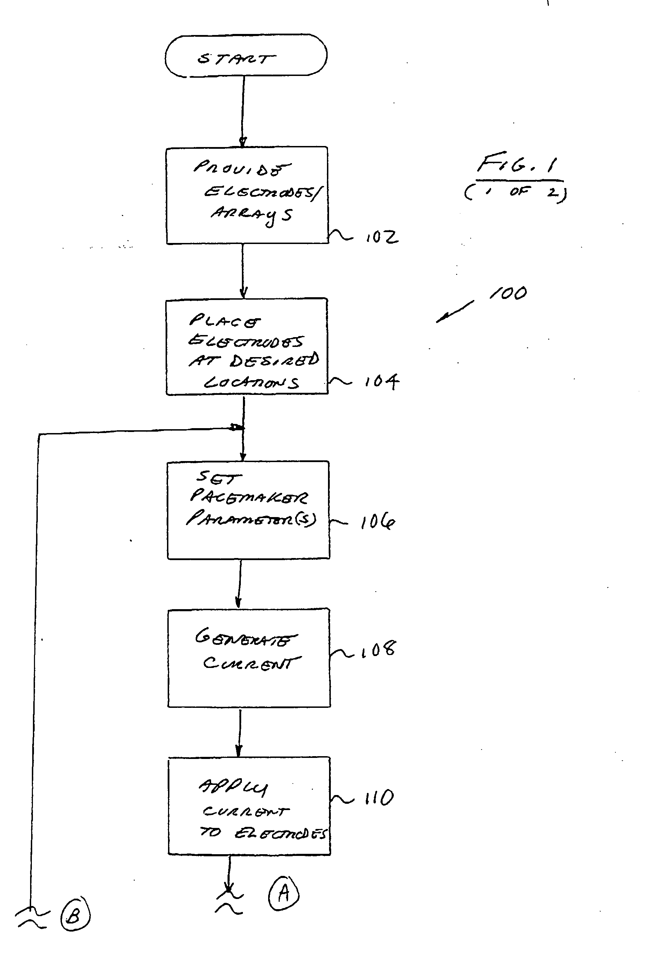

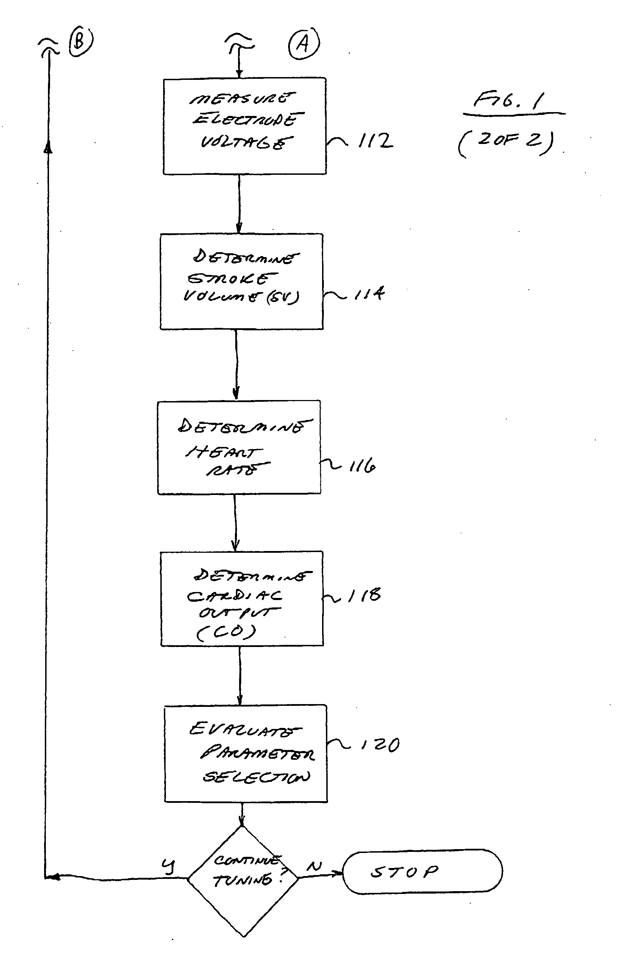

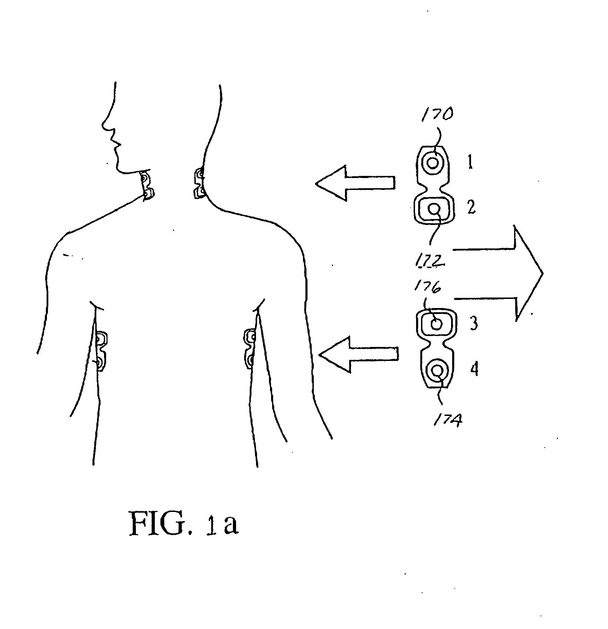

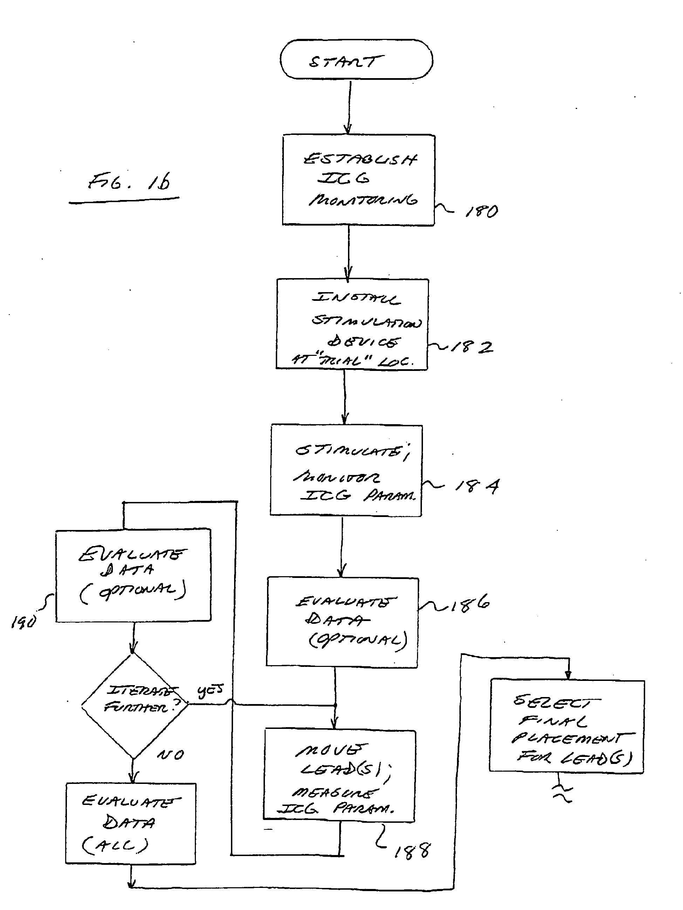

Referring now to Figs. 1-1a, the general methodology of non-invasively tuning an implantable stimulation device in a living subject according to the invention is described.

Fig. 1 illustrates the logical flow of the method of tuning the pacemaker based on stroke volume. As shown in Fig. 1, the method 100 generally comprises first providing a plurality of ICG electrodes or electrode "arrays" per step 102. Exemplary electrodes for this purpose are described in, *inter alia,* the aforementioned U.S. patent application Serial Nos. 09/613,183 entitled "Apparatus And Method For Determining Cardiac Output In A Living Subject" filed July 10, 2000, now U.S. Patent No. 6,636,754 issued October 21, 2003, and 09/903,473 entitled "Apparatus and Method for Determining Cardiac Output in a Living Subject" filed July 10, 2001, now U.S. Patent No. 6,602,201 issued August 5, 2003, as well as U.S. Design Patent Nos. D471,281 to Baura, et al. issued March 4, 2003 and entitled "Electrode for use on a living subject", D468,433 to Wagner, et al. issued January 7, 2003 and entitled "Electrode for use on a living subject", and D475,138 to Baura, et al issued on May 27, 2003 and also entitled "Electrode for use on a living subject". It will be recognized, however, that other types of electrodes (whether having single or multiple terminals) may be used consistent with the present invention.

The ICG electrodes/arrays are positioned at the desired locations above and below the thoracic cavity of the subject whose pacemaker is to be "tuned" per step 104, as illustrated in Fig. 1 a herein. In one embodiment of the method, these locations are chosen to be on the right and left sides of the abdomen of the subject, and the right and left sides of the neck. Other locations and/or combinations of arrays may be substituted with equal success. Optionally, pacemaker programmer ECG electrodes may also be positioned at the desired locations of right arm, left arm, right leg, and left leg. These pacemaker electrodes provide ECGs that the clinician may view on the pacemaker programmer display. However, because the ECG does not provide information on mechanical function, it cannot be used alone for pacemaker optimization.

Next, one or more settings or parameters (such as AV delay or VV skew) are set to desired values per step 106. These desired values may be determined according to any number of schemes, including without limitation (i) random selection (such as via a random or pseudorandom number generator which is limited within a range of physically reasonable values); (ii) predetermination based on, e.g., anecdotal or empirical data (such as prior clinical studies); or (iii) deterministic derivation based on measurement/analysis of one or more other physiologic or hemodynamic parameters, such as contemporaneous measurement of blood pressure, and the like. These settings or values may be resident within the pacemaker itself (such as being prestored within a storage device of the pacemaker), or otherwise "programmed into" the pacemaker at the time of tuning by an external device such as the aforementioned programmer wand. For example, the pacemaker may comprise an SoC embedded device with flash memory, wherein the memory is reprogrammed or reconfigured by the programmer wand via, e.g., modulated RF signals, thereby inserting user customized values. Alternatively, the pacemaker memory may be factory programmed with a plurality of different settings from which the programmer chooses. Myriad different schemes for programming a pacemaker with one or more desired values may be used consistent with the invention, such schemes being readily implemented by those of ordinary skill given the present disclosure.

Next, a substantially constant AC current is generated in step 108, and the current applied to the stimulation electrode terminal 170, 174 of each of the ICG electrode arrays in step 110. The voltage generated at the measurement electrode terminal 172, 176 of each electrode array is next measured in step 112. As previously discussed, this voltage is generally reduced from that applied to the stimulation electrode by virtue of the impedance of, *inter alia,* the thoracic cavity. Note that the measured voltage may be absolute, or relative (i.e., a differential voltage) as desired.

Next, in step 114, the cardiac stroke volume is determined from the measured voltage, using for example the Sramek-Bernstein relationship of Eqn. (2) below:

$$ SV = \frac{VEPT}{Z_o}\ LVET\ \frac{dZ\ (t)}{dt}_{min} , \qquad (2) $$

where *VEPT* = volume of electrically participating tissue; $Z_o$ = base impedance; *LVET* = left ventricular ejection time, the time interval during which the aortic valve is open; and $dZ(t)/dt_{min}$ is the maximum negative deflection of the impedance first time derivative. Lastly, per step 120, the stroke volume (or other ICG parameter) determined in step 114 (118) is used as at least a portion of the basis for evaluating the suitability of the parameter(s) selected on step 106.

While the foregoing embodiment of the method 100 is described in terms of measuring stroke volume (SV) as the basis of the suitability (optimization) evaluation, it will be recognized that other quantities or metrics may be used either alone or in combination with the foregoing as the basis of the evaluation. For example, an ICG parameter that is proportional to mitral regurgitation, the isovolumetric contraction time (IVCT), the isovolumetric relaxation time (IVRT), LVET, and/or stroke volume could be the basis of the optimization evaluation. Mitral regurgitation is characterized by incomplete closure of the mitral valve, causing blood to backflow into the left atrium when the left ventricle contracts. This backflow reduces the left ventricular stroke volume. A recent study by Breithardt, et al. (*JACC,* 41:765-70, 2003) demonstrated that an acute change in pacemaker stimulation (no stimulation vs. stimulation in right atrium, right ventricle, and left ventricle) can reduce MR by a mean of 48% in heart failure patients with left bundle branch block. The isovolumetric contraction time is the time interval between mitral valve closure and aortic valve opening. Typically 40 msec in normal subjects, IVCT is known to degrade to on the order 120 msec in left bundle branch block patients (Xiao, et al., *Br Heart J,* 69:166-173, 1993). The isovolumetric relaxation time is the time between aortic valve closure and mitral valve opening. As described above, LVET is the time interval during which the aortic valve is open. One ICG parameter that may be proportional to SV is the peak-to-peak amplitude of the time-varying impedance, $\Delta Z(t)$. MR, IVCT, IVRT, LVET and SV may all be quantified using echocardiography.

In another aspect of the invention, ICG is used to evaluate optimum lead placement for left ventricular pacing of the heart. Generally speaking, a right ventricular lead is relatively easy to place and stabilize. To pace the left side without puncturing the left side (high pressure) circulation, LV leads are typically placed in either the coronary sinus or pulmonary outflow tract, both accessible from the superior vena cava on the right side. Unfortunately, human anatomy is highly variable, especially through the coronary sinus, and the exact site of LV stimulation, and thus the efficiency of contraction, is highly variable on a patient-to-patient basis. Furthermore, due to this variability and the response of a particular subject, non-optimal behaviors arising from non-optimal lead placement during surgery may not be fully correctable through subsequent (non-invasive) adjustments to the stimulation device via the programmer. Accordingly, the present invention advantageously uses ICG to optimize lead placement as well as programmer settings. Here, the term "placement" may refer to not only the physical location of the lead, but also the suitability of the electrical characteristics established between the lead and the subject's tissue.

Specifically, as shown in the exemplary embodiment of Fig. 1b, the method generally comprises first fitting the subject with the ICG apparatus (i.e., stimulation and monitoring electrodes, etc.) so as to establish ICG monitoring (step 180). Next, the pacemaker or other stimulation device is installed within the patient (such as via surgery), with its electrical leads being disposed at a first "trial" locations (step 182). The ICG system is then used to monitor cardiac function during or subsequent to stimulation by the device so as to produce data (step 184), and the data associated with the first trial placement(s) optionally evaluated (step 186). The leads can then be moved *in situ* (step 188), and additional ICG data obtained and evaluated (step 190). Alternatively, the leads can be moved, data collected, leads moved, data collected, and so forth, with evaluation of the data occurring after all data has been collected. Optimal placement for the leads may then be ascertained from review of the data. Hence, the methodology of the present invention advantageously provides the user (e.g., surgeon) with a rapid and accurate means of locating stimulation device leads during the surgical procedure, as opposed to the prior art approaches which in effect comprise an "educated guess".

In a further application, ICG may be used to optimize atrial and/or ventricular electrostimulation. For example, programmer settings may be optimized to minimize the number of ectopic beats and occurrence of preventricular contractions, or to maximize ventricular capture.

*Apparatus*

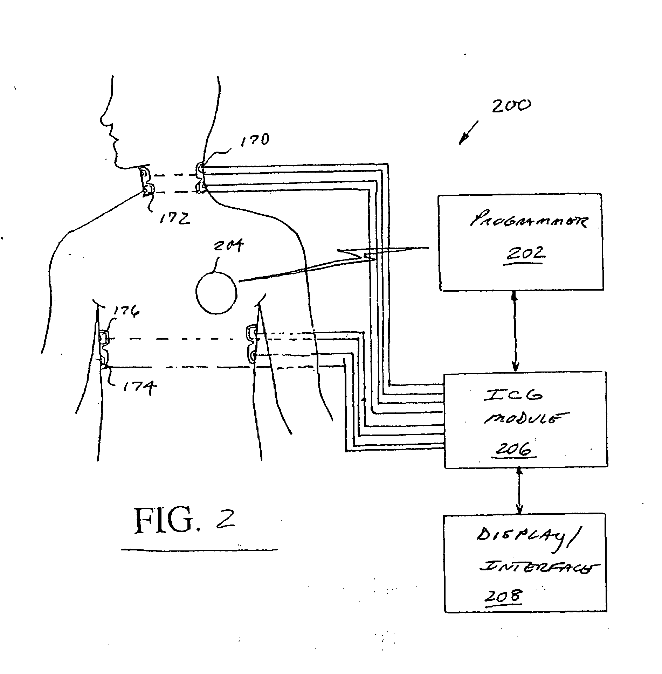

It will be appreciated that all or parts of the methodology 100 of Fig. 1 may be practiced iteratively in order to optimize the process of selecting parameters for (i.e., "tuning") the pacemaker. For example, in one exemplary configuration, the foregoing methodology is embodied in the hardware and software of an ICG device such as that described in U.S. 09/903, entitled "Apparatus and Method for Determining Cardiac Output in a Living Subject" filed July 10, 2001, now U.S. Patent No. 6,602,201 issued August 5, 2003. Fig. 2 illustrates an exemplary ICG module and pacemaker programmer configuration 200. Specifically, the user initiates an optimization routine (such as by choosing a particular programmer command on the programmer device 202). This optimization routine causes the programmer device to automatically program a first value (or set of values) into the pacemaker 204, and then obtain measurement(s) of stroke volume via the ICG module 206. Then, a second value or set of values is programmed into the pacemaker 204, a second SV measurement obtained, and so forth, until a desired number of values have been inserted and corresponding SV measurements obtained. At each new setting, the ICG module 206 stimulates with constant current, measures resulting voltage, and analyzes ICG waveform to determine ICG stroke volume and/or another ICG optimization variable.

After all or a subset of the desired settings have been programmed and the corresponding optimization variable measurements obtained, the ICG module 206 determines the setting that produced the most optimal case of the ICG optimization variable. Here, the phrase "most optimal case" is used generally to refer to any situation or series of events which are used as the basis for evaluating the sufficiency of tuning of the stimulation device. For example, in a simple case, optimization may comprise the maximal value of stroke volume. Alternatively, if mitral regurgitation was being monitored, the lowest value would be optimal. As yet another example, more sophisticated analysis or processing of the data may be used, such as where a plurality of data points (e.g., SV values collected over a given interval of time) are analyzed to determine statistical mean/median values, these derived statistical values being used directly or indirectly as the basis for evaluation. Consider the exemplary case where a plurality of SV or measurements obtained via ICG are analyzed for their standard deviation ($\sigma$) or variance ($\sigma^2$) , with these latter values being used to assess the acceptability of the measured values for subsequent use in tuning. Many different variants and permutations of the foregoing may be used consistent with the methodology 100 of Fig. 1.

It will be further appreciated that this determination need not necessarily be performed subsequent to the collection of all data, but rather may also be performed "on the fly", such as by evaluating the data collected to that point in the process, and selectively either adding new data to the collected data set or discarding data based on its relationship to the previously collected data. For example, in one embodiment, the ICG module 206 is programmed to (i) collect SV, etc. data associated with a first programmed setting, (ii) revise the setting(s) and measure the optimization parameters) again, and (iii) compare the values for the optimization parameter associated with the revised setting to that associated with the prior settings, and selectively retain or discard the new settings. If the SV value measured for the first setting(s) was 50 ml, and the value for the second setting was 60 ml, the second setting would be retained in a storage area (and the first discarded) since the second setting produced a higher magnitude of stroke volume. Similarly, if a third setting produced an even higher value of SV, the third setting(s) would be retained and the second (set) discarded. However, if the third setting produced a lower SV value, the third setting(s) would be discarded, and the second retained for ultimate comparison to a fourth value, and so forth. In this fashion, the pacemaker setting(s) producing higher values of the optimization parameter are retained, in effect "ratcheting" upward until the maximum value is reached. This approach ostensibly may reduce processing overhead and lag, since the optimization (comparative) computations are being performed concurrent with the setting iteration and ICG measurement process.

Numerous other approaches to evaluating the data may also be used, including multivariable analysis such as $2^k$ Factorial Design (i.e., looking at data for two or more optimization parameters, and determining the optimal pacemaker settings based on an algorithm which maximizes all of the parameters), or even fuzzy variable analysis of the type well known in the art and described in detail in the aforementioned co-pending applications and Patents.

It will also be recognized that variation in the one or more stimulation device parameters need not be sequential, uniform, or of constant direction, although each of the foregoing clearly may be used. For example, in one simple example, a first pacemaker value (e.g., AV delay) is varied by a constant amount or percentage in an increasing or decreasing direction at regular iteration intervals, the latter which may be measured in number of cardiac cycles. Alternatively, a non-constant amount or percentage may be used, such as when "fine tuning" using small increments of variation to find the optimal value after a more coarse evaluation has been conducted. As yet another alternative, a high/low approach may be used, wherein the value of the pacemaker parameter is varied in a first direction (e.g., an increase over the starting value), and then in a second direction (decrease compared to starting value), then a subsequent second increase, and so forth. As yet even another alternative, a first parameter may be varied according to any of the foregoing schemes, and then a second parameter varied according to the same or different scheme, which may then be followed by another optimization of the first parameter, and so forth. Hence, the present invention is in no way limited to any particular method or scheme for parameter variation and cardiac function measurement.

Furthermore, if two or more parameter values of the pacemaker produce similar or identical values of the monitored cardiac function (e.g., SV), then further processing may be performed so as to resolve the ambiguity. While it is feasible that such two or more values may be equally desirable for a given subject, there is most often some basis for differentiation or selection of one value from the other(s) as being optimal. In one embodiment of the present invention, ambiguity resolution is performed by evaluating a second parameter of interest. For example, if one of two values of W skew produces a higher IVCT (with identical stroke volume), the other value resulting in the lower heart rate may be chosen as being optimal. As another alternative, one such value may produce greater mitral regurgitation, and therefore be less optimal. In another embodiment, the presence of an ambiguity is used to generate a signal alerting the user/operator as to the presence of the ambiguity (as well as, optionally, other pertinent information), the user/operator then utilizing their expertise or an expert system to select the appropriate parameter value(s). It will be apparent to those of ordinary skill that while the foregoing is described in terms of a single parameter such as AV delay or VV skew, multivariable analysis and ambiguity resolution may be used consistent with the invention, the foregoing being merely illustrative of the broader concepts.

Conversely, the optimization process may be conducted with significant delay and/or at a remote processing facility. For example, the optimization may be performed on data streamed from the ICG module via a network interface (e.g., LAN, WAN, MAN, internet, hybrid fiber coax (HFC) w/ DOCSIS modem, etc.) to a remote processing node. In one exemplary variant, the streamed data is analyzed in light of historical data relating to the individual subject being evaluated, so as to place the current monitoring/evaluation session in the larger context of that subject's entire monitored history. Simultaneously (or alternatively), the remote processing may access data for other subjects; e.g., those having similar physiologic conditions, so that that the streamed data can be evaluated against norms or averages of larger populations, or against the data of another specific subject (or group of subjects, such as all patients in the database who have the same height, weight, and age, etc.). Using this remote processing mechanism, the streamed data may also be added to these historical databases. if desired. This "remote processing" approach has the advantage of allowing each remote processing station (i.e., ICG module and associated pacemaker programmer) to be quite "thin" in terms of software and database overhead, since each can readily access a large repository of relevant data without having to maintain it on-site. Furthermore, database administration is reduced, since a central "dial in" database is more readily maintained and updated as compared to a plurality of local databases. Attendant access control and/or encryption devices of the type well known in the data networking arts (e.g., RADIUS server, tunneled packets via VPN, etc.) may also be employed as desired to control access and avoid corruption of the transmitted data and remote database(s).

After analysis and evaluation, the results may be returned to the monitoring site (via the same network connection) to complete the tuning process. Given prevailing high bandwidth data connections and processing technology, it is feasible that the aforementioned remote processing could be completed in as little as a few seconds. Alternatively, if a specialist or other expert system is to be consulted, the remote site can signal the local monitoring site to insert a default set of pacemaker values during the interim period, thereby maintaining the subject in a stable physiologic condition until optimal pacemaker values are identified.

Once the optimized parameter (set) has been determined by the ICG module 206, it transmits this parameter to the pacemaker programming device 202 (or otherwise causes these values to be adopted by the pacemaker 204, such as from pacemaker on-board storage). The pacemaker 204 is then operated using the optimal settings until further tuning, if any, is required.

While the illustrated embodiment of Fig. 2 shows a wired data connection between the ICG module 206 and the programmer device 202 (i.e., a USB, RS-232, IEEE-1394 "Firewire", or comparable data port arrangement with a physical cable disposed between the devices), it is recognized that the data interface between these devices may be wireless as well, for example those commonly available and employing any number of communication protocols and frequency ranges including without limitation IEEE Std. 802.11, Bluetooth 2.4 GHz, Wireless Medical Telemetry Service (WMTS) medical band (608-614 MHz), 900 MHz or 2.4 GHz ISM bands, 5 GHz band, TM-UWB, and IrDA.

It will also be appreciated that the foregoing procedure is readily implemented by a single input from the user, i.e., so-called "one button optimization", thereby simplifying the process of tuning the pacemaker 204. Such functionality may be contained in the form of a GUI generated on display (e.g., by clicking on an icon, selecting a menu entry, selecting a sensitized area of a capacitive or touch screen having programmable "soft" function keys or SFKs, etc.) associated with the programmer device 202 or even a remote display/GUI device 208, or in the form of hardware/firmware as in a fixed function key (FFK). Alternatively, the user interface may be aural, such as in the form of a speech recognition system adapted to recognize the user's verbal commands and initiate the procedure. As yet another alternative, the optimization procedure may be made completely automatic, as where upon proper registration of the ICG module 206 and the programmer device 202 (and the pacemaker 204) such as via RF signals or other protocols occurring at connection and/or power-up of the device(s), the optimization procedure is initiated automatically. Furthermore, the optimization may be triggered upon the occurrence of a certain event, including for example the expiration of a given period of time from the last optimization, at a given time/date, upon detection of a certain artifact or waveform present in physiologic signals, etc. All such initiation/control functions are readily implemented by those of ordinary skill in the electronic and programming arts, and accordingly not described further herein.

Furthermore, the optimization algorithm previously described may be disposed entirely or at least partly within the ICG module 206 as opposed to the programmer device 202. For example, an SFK or FFK on the module may be used to initiate running of the algorithm on the ICG processor (not shown), with communication between the ICG module and the programmer being conducted

To maximize efficiency, the ICG module and pacemaker programmer device of the present embodiment do not transmit/receive concurrently (in effect being slotted according to a simple TDMA scheme), as the ICG and programmer carrier frequencies are often near each other (e.g., approximately 70 kHz vs. 100 kHz, respectively). However, it will be recognized that other types of spectral access and air interface schemes may be employed consistent with the invention to effectuate communications between the pacemaker 204 and the programmer device 202 without interference to the 70 kHz signals of the ICG module 206. For example, in one alternate embodiment, a direct sequence spread spectrum (DSSS) system is used with a pn (pseudo-noise) spreading code. As yet another alternative, an FHSS system with pseudorandom hop sequence is used. In another embodiment, an FDMA ("narrowband") approach is used, with GMSK "upshift" and "downshift" modulation used to encode data. In even another alternative, a time-modulated ultra-wide bandwidth (TM-UWB) approach is utilized. Myriad other arrangements well known in the RF arts may be used consistent with the present invention.

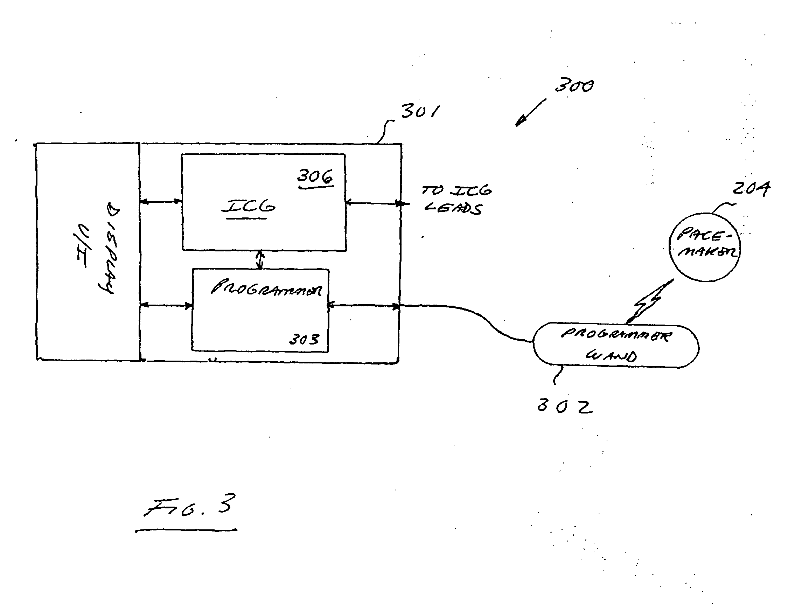

Fig. 3 illustrates another exemplary embodiment of the apparatus, wherein the aforementioned ICG module and pacemaker programmer device are physically integrated into a single device. Here, a standalone box 301 with programmer wand 302 and ICG module 306 are is provided, with communications between the programmer 303 and ICG module 306 being conducted internal to the box 301, thereby obviating the need for the aforementioned wired or wireless interfaces between the separate devices. Such internal communications may be effectuated using any number of techniques well known in the computer and electronic arts, including for example use of a PCI or cPCI bus architecture, RapidIO configuration, interprocessor communications, etc., which accordingly are not described further herein.

In another variant, the ICG functionality and processing are disposed within an existing pacemaker programmer housing structure (not shown), such as through the addition of an ICG card into the chassis of the programmer device. Conversely, the programmer functionality may be disposed within the ICG module via a peripheral. As yet another alternative, the programmer device may be embodied as a wireless "dongle" of the type well known in the data networking arts which mates with an existing port on the ICG modules, such as an RS-232, USB, IEEE Std. 1394, etc. interface. Common integrated or stand-alone monitor, display, and input devices may also be used. Many other possible physical manifestations of the invention are possible; hence, the present invention should in no way be considered limited to the exemplary embodiment described herein.

In another variant of the invention, the system (e.g., the ICG module 206, 306) is configured to provide the operator with a graphical representation of the analysis performed during optimization. In the exemplary embodiment, the relevant parameter associated with the ICG measurement and used as the basis for optimization (e.g., stroke volume) is displayed as a function of the values of the selected stimulation device parameter (e.g., AV delay, VV skew) or combinations thereof. Such graphical representation may comprise any number of well known display formats, such as bar, line, histogram, 3-D, contour, scatter plot, etc., as desired by the user. Hence, the graphical representation allows the operator to visualize the relationship(s) between ICG measurements and changes in pacemaker programming. In the exemplary embodiment, the user is also provided with a second graphical representation (e.g., icon, illumination, flashing signal, etc.) which indicates the optimal pacemaker setting, and the ability to accept or reject this setting in a "one button" context (thereby also inserting this setting into the pacemaker via the programming device). Hence, during a typical operational cycle, the system sequences through a series of AV/VV settings while measuring SV of the subject's heart; the various AV/VV parameter value(s) are displayed for the user (with or without the corresponding SV values), with the best or optimal pacemaker settings (i.e., those which produce the maximal SV) being annotated or highlighted for the user. The user then merely invokes acceptance or rejection of the "maximal" parameters, such as via a SFK, FFK, pull-down menu, audible voice command, or other user interface mechanism, at which point the system programs these settings into the pacemaker.

While not required, the ICG module 206, 306 of the present invention may also contain any number of different features to provide enhanced ICG functionality and accuracy, including for example the use of ICG electrodes with predetermined spacing as described in co-pending U.S. Application Serial No. 09/613,183, now U.S. Patent No. 6,636,754 issued October 21, 2003, previously referenced herein. Similarly, the automated selection of optimum ECG lead configuration as described in U.S. Application Serial No. 09/903,473 filed July 10, 2001 and entitled "Apparatus and Method for Determining Cardiac Output in a Living Subject", now U.S. Patent No. 6,602,201 issued August 5, 2003, may also be used.

Automated pacemaker spike detection methods and apparatus as described in U.S. Application Serial No. 10/329,129 filed December 24, 2002 and entitled "Method and Apparatus for Waveform Assessment", may also be used consistent with the invention in order to provide accurate spike detection in accordance with the methodologies disclosed herein. Specifically, the subject's ECG waveforms are analyzed to identify atrial and ventricular pacing spikes. When these spikes are detected, they may substitute as Q points during definition of the $\Delta Z$ search interval for B, C, and X points in a thoracic impedance waveform. Such searches may be conducted using, for example, the wavelet transform model described in co-pending and co-owned U.S. Patent No. 6,561,986. Pacing spike detection in the aforementioned patent incorporates some aspects of artificial intelligence, in that it is desirable to detect spikes of varying amplitude, with variable time delays between the A and V spikes, in the presence of noise also having a variable amplitude. This is accomplished using a golden section search optimization technique, in conjunction with a fuzzy model. The golden section search identifies spikes or other artifacts based primarily on their shape as opposed to amplitude or other criteria, thereby significantly increasing the robustness of the detection algorithm. A fuzzy model is used to calculate the noise threshold, in that a pacemaker spike must be distinguishable from its surrounding noise. Because the spike and noise amplitudes vary with each subject, the noise threshold is calculated in one embodiment during the beginning of a monitoring session, or immediately after a lead change. This noise threshold is then used as an input to the AV spike detector. Table 1 below provides a summarization of the exemplary parameters related to pacing spike detection and cardiac output determination.

Table 1

| A | Reference to right atrium |
|---|---|
| B | Sample signifying initial aortic valve opening. |
| C | Sample signifying maximum deflection in the inverse of the first time derivative of $\Delta Z$ |
| $\Delta Z$ | Time-varying portion of the impedance signal |
| ECG | Electrocardiogram |
| $k$ | Discrete time sample |
| Q | Sample signifying initial left ventricular depolarization |
| V | Reference to right ventricle |
| X | Sample signifying aortic valve closure |

The ECG fiducial point detection may also optionally be optimized for arrhythmia detection (described in co-pending application Serial No. 10/393,544 filed January 17, 2001 and entitled "Apparatus and Method for Defibrillation of a Living Subject", may be used. Specifically, the 10/393,544 application describes an approach for determination of shockable and nonshockable rhythms, including VT and SVT, determining if significant and pulsatile cardiac output (blood flow through the heart) is present with each heartbeat. Pacing spike detection is implemented to prevent misclassification of pacing spikes as R points. A wavelet algorithm for efficient R point detection during arrhythmias is also used in conjunction with the foregoing. A wavelet transform is a time-scale representation of an input signal that is obtained by filtering the signal with a wavelet or scaling function at a particular scale. Various wavelets and scaling functions are utilized as part of the invention to emphasize certain features of interest associated with the input impedance and/or ECG waveforms obtained from electrodes positioned on the subject's thorax. The resulting emphasized feature in each wavelet transform is then detected to obtain a fiducial point (e.g., B, C, X for the impedance waveform, and R for the ECG waveform). By virtue of its transformation to the time-scale domain, this wavelet method is more resistant to noise artifact than empirical waveform processing in the time domain. Furthermore, no absolute thresholds are used for R, B, C, or X point detection in the exemplary embodiment, which increases the ability of this algorithm to generalize among waveforms from the cardiac patient population. The use of a decision model not constrained to discrete values or absolute thresholds (e.g. a fuzzy logic model) ensures that the decision module is capable of such generalization. With efficient beat detection, the variability of B point and X point samples can also advantageously be determined with higher certainty.

In the exemplary embodiment, ECG and ICG waveforms are acquired from the hardware, with subsequent software processing performed to analyze and extract the desired information. In general, the method of the present embodiment comprises first obtaining signals from the subject containing information relating to the cardiac function of the subject. Next, these signals are analyzed in terms of multiple parameters. Information generated by this analysis is then used as input to a decision process or used for other purposes.

Specifically, in this embodiment, the ECG is first analyzed for pacing spikes or R points. If pacing spikes are detected, then the $\Delta Z$ and dZ/dt waveforms are parsed into individual beats according to a first criterion. In one variant, parsing into individual beats of $\Delta Z$ and dZ/dt occurs based on the pacing spikes. If pacing spikes are not detected, then the $\Delta Z$ waveforms are parsed according to a second criterion, such as for example detected R points. Within each beat, a plurality of fiducial points (e.g., two), such as the B (aortic valve opening) and X (aortic valve closing) points, are determined. Over several beats, the standard deviations of the fiducial point sample locations are calculated. These standard deviations are input to a decision process; when the standard deviations meet certain criteria (such as, for example, the values of the standard deviations being sufficiently large in magnitude), the heart may be unable to pump blood in a coordinated fashion, as the aortic valve openings and closures are highly variable.

In the exemplary embodiment of the present invention, the ECG waveform obtained from the subject (or intermediary device) is processed in the ECG "module" to detect artifacts. As used herein, the term "module" may comprise a physical device or component within the ICG module, or alternatively a "virtual" module, such as in the case of where the signal processing performed by the module is distributed across a number of different components or software processes, and/or distributed temporally. Hence, the term module should in no way be considered limiting with respect to any particular configuration, arrangement, or sequence.

One such exemplary artifact detected by the ECG module comprises pacing spikes, with detection according to the methodologies set forth in co-owned and co-pending U.S. patent application Serial No. 10/329,129 filed December 24, 2002 and entitled "Method and Apparatus for Waveform Assessment". Specifically, if one or more (e.g., two) pacing spikes are present between QRS complexes, the most recent spike is used to parse the next beats of $\Delta Z$ and dZ/dt. The parsed $\Delta Z$ and dZ/dt beats are transmitted for further processing. If a spike is not present, then R point detection is conducted, with the detected R point used to parse the next beats.

Exemplary methods of R point detection are described in e.g., U.S. Patent No. 6,561,986 referenced above, with the exception that these methods are ideally adapted to detect arrhythmic R points with enhanced accuracy. Specifically, in one variant, the R point detection methodology is adapted in four (4) aspects. First, rather than a single threshold for QRS complex detection, two thresholds are used. A first threshold (such as for example 10% of the max value of the preceding 300 samples) is provided, and a second threshold (such as for example 27.5% of the previous QRS complex amplitude in the wavelet test value signal) is also provided. In one variant, the larger of the two values is used for beat detection, although it will be recognized that more sophisticated analytical methods relating to the two (or more) thresholds may be implemented, such methods being readily implemented by those of ordinary skill provided the present disclosure.

Second, the maximum value window for detection of a QRS complex is set at 40 samples. This number may be varied, however, based on the desired application and other factors.

Third, rather than only looking for the R point in the time domain, the instant embodiment first isolates the R point in the wavelet test value signal in a sample "window" (e.g., 50 samples) around the QRS complex location. Once this isolation is performed, the maximum absolute value of the ECG in another sample window (e.g., 7 samples) around this point is marked as the R point.

Lastly, the next sample to begin looking for a QRS complex location is set to the falling edge of the QRS complex window. This is the first sample that is less than the maximum value of the wavelet test value window that contains the QRS complex. Specifically, in the illustrated embodiment, the WT_max signal (the signal having the maximum value of the previous 40 samples) is used to establish an upper bound. This in effect generates a "box" in the signal around a QRS complex, where the left-hand corner of the box comprises the QRS complex location. When the first sample having a smaller magnitude than that corresponding to the top (upper bound) of the box is encountered, the search for another QRS complex is performed. This first smaller magnitude sample is referred to as the "falling edge", because the signal starts declining towards 0 until the baseline between beats is reached. The following parameters are also specified in the exemplary embodiment:

1) *Falling_edge(m)*- QRS window falling edge:

$$m \in [0,...,M], \ M+1 = \text{number of QRS complexes}$$

The offset of the QRS window, *Falling_edge(m),* is designated as the first sample after *Rising_edge(m)* that satisfies Eqn. (3):

$$w_T(k) > w_T(k+1) \tag{3}$$

2) $x_{Start}(k)$ - Next start sample:

$x(k)$ is the sample number to begin looking for next QRS complex. The next start sample shall be the first sample after the *Falling_edge(m),* or $x_{Start}(k) = Falling\_edge(m)+1$.

It will recognized that the foregoing features including use of predetermined electrode configuration, use of pacing spike detection, use of a wavelet algorithm, use of a non-crisp decision model, such as a fuzzy model, may be employed alone or in varying combinations within any method or apparatus under the present invention. Hence, depending on the desired level of accuracy and functionality for a given application or instrument, none or more of these features may be utilized. Such features may even be made optional or user-selectable if desired. For example, the use of pacing spike detection, wavelet processing, and non-crisp decision-making may afford sufficient accuracy in the absence of automated ECG lead selection. The invention should therefore in no way be considered to be limited to specific combinations or the aggregation of all such features, but rather may be more broadly practiced with or without these features.

Also, it will be appreciated that while the ICG (and ECG) signal processing set forth herein is described primarily in terms of software algorithms, performance of at least portions of these analysis may be performed in hardware or firmware, such as for example in preconfigured logic gates (e.g., FPGAs or ASICs) or DSP front-end or back-end analog processing. The present invention should therefore not be considered to be limited to software-based processing.

It will be further recognized that while certain aspects of the invention have been described in terms of a specific sequence of steps of a method, these descriptions are only illustrative of the broader methods of the invention, and may be modified as required by the particular application. Certain steps may be rendered unnecessary or optional under certain circumstances. Additionally, certain steps or functionality may be added to the disclosed embodiments, or the order of performance of two or more steps permuted. All such variations are considered to be encompassed within the invention disclosed and claimed herein.

While the above detailed description has shown, described, and pointed out novel features of the invention as applied to various embodiments, it will be understood that various omissions, substitutions, and changes in the form and details of the device or process illustrated may be made by those skilled in the art without departing from the invention. The foregoing description is of the best mode presently contemplated of carrying out the invention. This description is in no way meant to be limiting, but rather should be taken as illustrative of the general principles of the invention. The scope of the invention should be determined with reference to the claims.

**Claims**

1. Apparatus (202) for use with a pacemaker (204), comprising thoracic impedance cardiography apparatus including means adapted to determine at least one aspect of cardiac function under varied conditions of stimulation of the heart produced by said pacemaker, and means adapted to tune said pacemaker based at least in part on said determination;
   wherein said apparatus is adapted for use externally to a living subject,
   whereby said determination is based on the impedance measured before and after variation of the conditions of stimulation.

2. The apparatus of Claim 1, further comprising control apparatus adapted to control said conditions of said stimulation device.

3. The apparatus of Claim 2, wherein said control apparatus comprises a programming device adapted to vary one or more settings of said pacemaker device *in situ.*

4. The apparatus of Claim 3, wherein said act of varying comprises transmitting data from said programming device to said pacemaker device via electromagnetic energy.

5. The apparatus of Claim 2, wherein said control apparatus is adapted to iterate said conditions through at least two different settings, said cardiography apparatus measuring said at least one aspect for each of said at least two iterations.

6. The apparatus of Claim 1, wherein said at least one aspect is selected from stroke volume.

7. The apparatus of Claim 1, wherein said cardiography apparatus comprises at least one set of electrodes having predetermined inter-electrode spacing.

8. The apparatus of Claim 1, wherein said cardiography apparatus comprises wavelet transform signal processing.

9. The apparatus of Claim 8, wherein said wavelet transform signal processing is used to identify at least one fiducial point within an impedance waveform

10. The apparatus of Claim 8, wherein said wavelet transform signal processing is used to identify at least one fiducial point within an ECG waveform.

11. The apparatus of Claim 1, wherein said cardiography apparatus comprises pacing spike detection.

12. The apparatus of Claim 11, wherein said pacing spike detection is accomplished at least in part via a fuzzy model.

13. The apparatus of Claim 2, wherein said cardiography apparatus comprises a discrete impedance cardiography (ICG) module in data communication with said control apparatus.

14. The apparatus of Claim 1, wherein said cardiography apparatus and said control apparatus operate according to a substantially time divided or multiplexed scheme.

15. The apparatus of Claim 1, wherein said cardiography apparatus comprises substantially automated ECG lead selection.

16. The apparatus of Claim 1, wherein said varied conditions of stimulation comprise at least one condition selected from the group consisting of (i) AV delay, and (ii) VV skew.

17. The apparatus of Claim 1, wherein determining under varied conditions is initiated through a single user action.

18. The apparatus of Claim 1, wherein said varied conditions of stimulation originate from a stimulation source adapted to produce a stimulation current.

19. The apparatus of Claim 18, further comprising:

    a plurality of electrodes adapted for use on a living subject, at least a portion of said electrodes being further adapted to apply said stimulation current to said subject.

20. The apparatus of Claim 19, further comprising:

    a data interface adapted to transmit data between said apparatus and a pacemaker controller configured to control at least one parameter associated with a pacemaker.

21. The apparatus of Claim 20, further comprising:

    a signal processing apparatus operatively coupled to at least a portion of said electrodes and said data interface, said signal processing apparatus adapted to coordinate evaluation of signals obtained from said at least portion of electrodes with changes in said at least one parameter of said pacemaker in order to identify an optimal value thereof.

22. The apparatus of Claim 18, further comprising:

at least one electrode interface adapted to communicate electrical signals with a plurality of electrodes used on a living subject, at least a portion of said electrodes being adapted to apply said stimulation current to said subject.

23. The apparatus of Claim 22, further comprising;
a data interface adapted to transmit data between said apparatus and a controller configured to control at least one parameter associated with a cardiovertor-defibrillator.

24. The apparatus of Claim 23, further comprising:

a signal processing apparatus operatively coupled to at least a portion of said electrode and data interfaces, said signal processing apparatus adapted to coordinate evaluation of signals obtained from said at least portion of electrodes with changes in said at least one parameter of said defibrillator in order to identify an optimal value thereof.

**Patentansprüche**

1. Gerät (202) zur Verwendung mit einem Schrittmacher (204), der thorakales Impedanz-Kardiografiegerät einschließlich Mitteln umfasst, die angepasst sind mindestens einen Aspekt von Herzfunktion unter unterschiedlichen Stimulationszuständen zu bestimmen, die von besagtem Schrittmacher produziert werden und Mittel umfasst, die angepasst sind, besagten Schrittmacher mindestens teilweise auf besagter Bestimmung beruhend einzustellen; wobei besagtes Gerät zur externen Verwendung an einem lebenden Subjekt angepasst ist, wobei besagte Bestimmung auf der Impedanz beruht, die vor und nach Variation der Stimulierungszustände gemessen wurde.

2. Gerät nach Anspruch 1, das weiter Steuergerät umfasst, das angepasst ist, besagte Zustände der besagten Stimulationsvorrichtung zu steuern.

3. Gerät nach Anspruch 2, wobei besagtes Steuergerät eine Programmiervorrichtung umfasst, die angepasst ist, eine oder mehrere Einstellungen der besagten Schrittmachervorrichtung in situ zu variieren.

4. Gerät nach Anspruch 3, wobei besagte Variierhandlung das Senden von Daten von besagter Programmiervorrichtung zur besagten Schrittmachervorrichtung über elektromagnetische Energie umfasst.

5. Gerät nach Anspruch 2, wobei besagtes Steuergerät angepasst ist, besagte Zustände durch mindestens zwei verschiedene Einstellungen zu wiederholen, wobei besagtes Kardiografiegerät mindestens einen Aspekt für jede der besagten mindestens zwei Wiederholungen misst.

6. Gerät nach Anspruch 1, wobei mindestens ein Aspekt vom Schlagvolumen selektiert wird.

7. Gerät nach Anspruch 1, wobei besagtes Kardiografiegerät mindestens einen Satz Elektroden mit vorbestimmten Elektrodenzwischenraum umfasst.

8. Gerät nach Anspruch 1, wobei besagtes Kardiografiegerät Wavelet-Umformsignalverarbeitung umfasst.

9. Gerät nach Anspruch 8, wobei besagte Wavelet-Umformsignalverarbeitung verwendet wird, mindestens einen Bezugspunkt innerhalb einer Impedanzwellenform zu identifizieren.

10. Gerät nach Anspruch 8, wobei besagte Wavelet-Umformsignalverarbeitung verwendet wird, mindestens einen Bezugspunkt innerhalb einer EKG-Wellenform zu identifizieren.

11. Gerät nach Anspruch 1, wobei besagtes Kardiografiegerät Schrittmacher-Spitzenerkennung umfasst.

12. Gerät nach Anspruch 11, wobei besagte Schrittmacher-Spitzenerkennung mindestens teilweise über eine Fuzzy-Modell erzielt wird.

13. Gerät nach Anspruch 2, wobei besagtes Kardiografiegerät ein diskretes Impedanz-Kardiografiemodul (ICG) in Datenkommunikation mit besagtem Steuergerät umfasst.

14. Gerät nach Anspruch 1, wobei besagtes Kardiografiegerät und besagtes Steuergerät nach einem im Wesentlichen zeitgeteilten oder gemultiplexten Plan arbeiten.

15. Gerät nach Anspruch 1, wobei besagtes Kardiografiegerät eine im Wesentlichen automatisierte EKG geführte Selektion umfasst.

16. Gerät nach Anspruch 1, wobei besagte unterschiedlichen Stimulationszustände mindestens einen Zustand umfassen, der aus der Gruppe ausgewählt ist, die aus (i) AV-Verzögerung und (ii) VV-Skew besteht.

17. Gerät nach Anspruch 1, wobei Bestimmung unter unterschiedlichen Zuständen durch eine einzelne Nutzermaßnahme initiiert wird.

18. Gerät nach Anspruch 1, wobei besagte unterschiedlichen Stimulationszustände von einer Stimulationsquelle herrühren, die angepasst ist, einen Stimulationsstrom zu produzieren.

19. Gerät nach Anspruch 18, das weiter umfasst:

Eine Mehrheit von Elektroden, die zur Verwendung an einem lebenden Subjekt angepasst sind, wobei mindestens ein Teil besagter Elektroden weiter angepasst ist, den besagten Stimulationsstrom auf besagtes Subjekt anzuwenden.

20. Gerät nach Anspruch 19, das weiter umfasst:

Eine Datenschnittstelle, die angepasst ist, Daten zwischen besagtem Gerät und einem Schrittmachersteuergerät zu übertragen, das konfiguriert ist, mindestens einen Parameter zu steuern, der mit einem Schrittmacher assoziiert ist.

21. Gerät nach Anspruch 20, das weiter umfasst:

Ein Signalverarbeitungsgerät, das funktionsfähig mindestens an einen Teil besagter Elektroden und besagter Datenschnittstelle gekoppelt ist, wobei besagtes Signalverarbeitungsgerät angepasst ist, Auswertung von Signalen zu koordinieren, die von besagtem mindestens einen Teil von Elektroden mit Veränderungen in besagtem mindestens einen Parameter von besagtem Schrittmacher erhalten wurden, um einen optimalen Wert davon zu identifizieren.

22. Gerät nach Anspruch 18, das weiter umfasst:

Mindestens eine Elektrodenschnittstelle, die angepasst ist, elektrische Signale mit einer Mehrheit von Elektroden zu kommunizieren, die an einem lebenden Subjekt verwendet werden, wobei mindestens ein Teil von besagten Elektroden angepasst ist, besagten Stimulationsstrom auf besagtes Subjekt anzuwenden.

23. Gerät nach Anspruch 22, das weiter umfasst:

Eine Datenschnittstelle, die angepasst ist, Daten zwischen besagtem Gerät und einem Steuergerät zu übertragen, das konfiguriert ist, mindestens einen Parameter zu steuern, der mit einem Kardiovertor-Defibrillator assoziiert ist.

24. Gerät nach Anspruch 23, das weiter umfasst:

Ein Signalverarbeitungsgerät, das funktionsfähig mindestens an einen Teil besagter Elektroden und besagter Datenschnittstellen gekoppelt ist, wobei besagtes Signalverarbeitungsgerät angepasst ist, Auswertung von Signalen zu koordinieren, die von besagtem mindestens einen Teil von Elektroden mit Veränderungen in besagtem mindestens einen Parameter von besagtem Defibrillator erhalten wurden, um einen optimalen Wert davon zu identifizieren.

## Revendications

1. Appareil (202) destiné à être utilisé avec un stimulateur cardiaque (204), comportant un appareil de cardiographie par impédance thoracique comprenant un moyen adapté pour déterminer au moins un aspect de fonction cardiaque dans des conditions variées de stimulation du coeur produite par ledit stimulateur cardiaque et un moyen adapté pour régler ledit stimulateur cardiaque en fonction au moins en partie de ladite détermination ;
   dans lequel ledit appareil est adapté pour être utilisé de manière externe à un sujet vivant,
   ce par quoi ladite détermination est fonction de l'impédance mesurée avant et après la variation des conditions de stimulation.

2. Appareil selon la revendication 1, comportant par ailleurs un appareil de contrôle adapté pour contrôler lesdites conditions dudit dispositif de stimulation.

3. Appareil selon la revendication 2, dans lequel ledit appareil de contrôle comporte un dispositif de programmation adapté pour varier un ou plusieurs réglages dudit dispositif stimulateur cardiaque *in situ.*

4. Appareil selon la revendication 3, dans lequel ladite action consistant à varier comporte la transmission de données en provenance dudit dispositif de programmation audit dispositif stimulateur cardiaque par le biais d'une énergie électromagnétique.

5. Appareil selon la revendication 2, dans lequel ledit appareil de contrôle est adapté pour itérer lesdites conditions par au moins deux réglages différents, ledit appareil de cardiographie mesurant ledit au moins un aspect pour chacune desdites au moins deux itérations.

6. Appareil selon la revendication 1, dans lequel ledit au moins un aspect est sélectionné parmi un volume systolique.

7. Appareil selon la revendication 1, dans lequel ledit appareil de cardiographie comporte au moins un ensemble d'électrodes ayant un espace prédéterminé entre les électrodes.

8. Appareil selon la revendication 1, dans lequel ledit appareil de cardiographie comporte un traitement de signaux par transformation en ondelettes.

9. Appareil selon la revendication 8, dans lequel ledit traitement de signaux par transformation en ondelettes est utilisé pour identifier au moins un point de calibrage à l'intérieur d'une forme d'onde de l'impédance.

10. Appareil selon la revendication 8, dans lequel ledit traitement de signaux par transformation en ondelettes est utilisé pour identifier au moins un point de calibrage à l'intérieur d'une forme d'onde de l'EGC.

11. Appareil selon la revendication 1, dans lequel ledit appareil de cardiographie comporte une détection de pointes de stimulation.

12. Appareil selon la revendication 11, dans lequel ladite détection de pointes de stimulation est réalisée au moins en partie par le biais d'un modèle flou.

13. Appareil selon la revendication 2, dans lequel ledit appareil de cardiographie comporte un module de cardiographie d'une impédance discrète (ICG) en communication de données avec ledit appareil de contrôle.

14. Appareil selon la revendication 1, dans lequel ledit appareil de cardiographie et ledit appareil de contrôle opèrent selon un schéma dans une large mesure réparti dans le temps ou multiplexé.

15. Appareil selon la revendication 1, dans lequel ledit appareil de cardiographie comporte une sélection dans une large mesure automatisée des dérivations d'ECG.

16. Appareil selon la revendication 1, dans lequel lesdites conditions variées de stimulation comportent au moins une condition sélectionnée parmi le groupe constitué (i) du délai trio-ventriculaire, et (ii) de l'asymétrie biventriculaire.

**17.** Appareil selon la revendication 1, dans lequel la détermination dans des conditions variées est initialisée par le biais de l'action d'un seul utilisateur.

**18.** Appareil selon la revendication 1, dans lequel lesdites conditions variées de stimulation proviennent d'une source de stimulation adaptée pour produire un courant de stimulation.

**19.** Appareil selon la revendication 18, comportant par ailleurs :

une pluralité d'électrodes adaptées pour être utilisées sur un sujet vivant, au moins une portion desdites électrodes étant par ailleurs adaptées pour appliquer ledit courant de stimulation sur ledit sujet.

**20.** Appareil selon la revendication 19, comportant par ailleurs :

une interface de données adaptée pour transmettre des données entre ledit appareil et un contrôleur de stimulateur cardiaque configuré pour contrôler au moins un paramètre associé à un stimulateur cardiaque.

**21.** Appareil selon la revendication 20, comportant par ailleurs :

un appareil de traitement de signaux couplé de manière opérationnelle sur au moins une portion desdites électrodes et de ladite interface de données, ledit appareil de traitement de signaux étant adapté pour coordonner l'évaluation des signaux obtenus en provenance de ladite au moins une portion des électrodes en fonction des changements dans ledit au moins un paramètre dudit stimulateur cardiaque afin d'identifier une valeur optimale de celui-ci.

**22.** Appareil selon la revendication 18, comportant par ailleurs :

au moins une interface d'électrodes adaptée pour communiquer des signaux électriques, une pluralité d'électrodes étant utilisées sur un sujet vivant, au moins une portion' desdites électrodes étant adaptée pour appliquer ledit courant de stimulation sur ledit sujet.

**23.** Appareil selon la revendication 22, comportant par ailleurs :

une interface de données adaptée pour transmettre des données entre ledit appareil et un contrôleur configuré pour contrôler au moins un paramètre associé à un défibrillateur à synchronisation automatique.

**24.** Appareil selon la revendication 23, comportant par ailleurs :

un appareil de traitement de signaux couplé de manière opérationnelle sur au moins une portion desdites électrodes et de ladite interface de données, ledit appareil de traitement de signaux étant adapté pour coordonner l'évaluation des signaux obtenus en provenance de ladite au moins une portion des électrodes en fonction des changements dans ledit au moins un paramètre dudit défibrillateur afin d'identifier une valeur optimale de celui-ci.

START

PROVIDE
ELECTRODES/
ARRAYS ～ 102

PLACE
ELECTRODES
AT DESIRED
LOCATIONS ～ 104

SET
PACEMAKER
PARAMETER(S) ～ 106

GENERATE
CURRENT ～ 108

APPLY
CURRENT
TO ELECTRODES ～ 110

FIG. 1
( 1 OF 2 )

100

Ⓐ

Ⓑ

17

B

A

MEASURE
ELECTRODE
VOLTAGE — 112

FIG. 1
(2 OF 2)

DETERMINE
STROKE
VOLUME (SV) — 114

DETERMINE
HEART
RATE — 116

DETERMINE
CARDIAC
OUTPUT
(CO) — 118

EVALUATE
PARAMETER
SELECTION — 120

Y ◇ CONTINUE
TUNING? ◇ N → STOP

FIG. 1a

Fig. 16

```
              ┌──────────────┐
              │    START     │
              └──────┬───────┘
                     │
                     ▼
              ┌──────────────┐
              │  ESTABLISH   │
              │     ICG      │
              │  MONITORING  │ ~ 180
              └──────┬───────┘
                     │
                     ▼
              ┌──────────────┐
              │   INSTALL    │
              │ STIMULATION  │
              │   DEVICE     │ ~ 182
              │ AT "TRIAL" LOC.│
              └──────┬───────┘
                     │
                     ▼
              ┌──────────────┐
              │  STIMULATE;  │
              │   MONITOR    │
              │ ICG PARAM.   │ ~ 184
              └──────┬───────┘
```

EVALUATE DATA (OPTIONAL)  190

EVALUATE DATA (OPTIONAL)  ~ 186

ITERATE FURTHER?   yes   no

MOVE LEAD(S); MEASURE ICG PARAM.  188

EVALUATE DATA (ALL)

SELECT FINAL PLACEMENT FOR LEAD(S)

FIG. 2

EP 1 484 083 B1

Fig. 3

EP 1 484 083 B1